# EUROPÄISCHE PATENTSCHRIFT

(11) **EP 4 175 854 B1**
(45) Veröffentlichungstag und Bekanntmachung des Hinweises auf die Patenterteilung: **26.03.2025**
(21) Anmeldenummer: 21745917.1
(22) Anmeldetag: 28.06.2021
(51) Int. Cl.: B60R 7/04, A61L 2/10, B60R 11/00

(54) **ABLAGEFACH FÜR EIN FAHRZEUG**
STORAGE COMPARTMENT FOR A VEHICLE
COMPARTIMENT DE STOCKAGE POUR UN VÉHICULE

(30) Priorität: 06.07.2020 DE 102020004058
(43) Veröffentlichungstag der Anmeldung: 10.05.2023
(73) Patentinhaber: Mercedes-Benz Group AG, 70372 Stuttgart (DE)
(72) Erfinder: MAIER, Valentin, 75245 Neulingen (DE); STAMP, Benno, 70619 Stuttgart (DE); MENZEL, Christoph, 70184 Stuttgart (DE)
(74) Vertreter: Novagraaf Group
(86) Internationale Anmeldenummer: PCT/EP2021/067688
(87) Internationale Veröffentlichungsnummer: WO 2022/008282

(56) Entgegenhaltungen:
- KR-A- 20150 030 527
- KR-A- 20150 074 359
- US-B1- 10 189 414

## Beschreibung

Die Erfindung betrifft ein Ablagefach für ein Fahrzeug mit einer integrierten Desinfektionsvorrichtung mit zumindest einer UV-C-Strahlung emittierenden Lichtquelle gemäß Oberbegriff des Patentanspruchs 1.

Die KR 2015 0074359 A offenbart ein gattungsgemäßes Ablagefach für ein Fahrzeug mit einer integrierten Desinfektionsvorrichtung mit zumindest einer UV-C-Strahlung emittierenden Lichtquelle und mit einer Auflage zur Auflage und Desinfektion eines Gegenstandes. Die Auflage weist eine die UV-C-Strahlung der zumindest einen Lichtquelle reflektierende Oberflächenstruktur auf, die ausgebildet ist, dass die reflektierte UV-C-Strahlung eine der Auflage zugewandte Seite des Gegenstandes bestrahlt, wenn ein Gegenstand aufgelegt ist, der nicht auf dem Boden der tiefsten Aufnahmenut der Auflage aufliegt. Nur dann hat die der Auflage zugewandte Seite des abgelegten Gegenstandes einen Abstand zu einem tiefsten Punkt der Oberflächenstruktur. Die Auflage kann eine Trägerstruktur umfassen, auf welcher eine Reflexionsschicht angeordnet ist. Die Trägerstruktur weist eine gestufte Nut bzw. Vertiefungen mit unterschiedlicher Längs- und Quererstreckung auf, so dass unterschiedlich große Gegenstände aufgenommen werden können. Eine ähnliche Ausgestaltung eines Ablagefachs ist aus der KR 10 2015 0030527 A bekannt.

Die US 10 189 414 B1 zeigt eine Ablagefach in einem Kraftfahrzeug, wobei das Ablagefach einen Boden aufweisen kann, der im Querschnitt trapezförmig ausgebildet ist und somit Erhebungen und Senken aufweist.

Aus der KR 10 1517 694 B1 ist ein Ablagefach für ein Fahrzeug bekannt, wobei das Ablagefach ein kabelloses Ladegerät und LED-Module an zwei Seiten des Gehäuses aufweist. Weiterhin ist das Ablagefach in X-Richtung verschiebbar ausgebildet Die LED-Module sind derart ausgebildet, dass ein in der Auflage abgelegter Gegenstand desinfiziert werden kann.

Der Erfindung liegt die Aufgabe zugrunde, ein gattungsgemäßes Ablagefach für ein Fahrzeug mit einer optimierten integrierten Desinfektionsvorrichtung anzugeben.

Die Aufgabe wird erfindungsgemäß durch ein Ablagefach gelöst, welches die in Anspruch 1 angegebenen Merkmale aufweist.

Vorteilhafte Ausgestaltungen der Erfindung sind Gegenstand der Unteransprüche.

Ein Ablagefach für ein Fahrzeug umfasst eine integrierte Desinfektionsvorrichtung mit zumindest einer UV-C-Strahlung emittierenden Lichtquelle. Es ist vorgesehen, dass eine Auflage zur Auflage und Desinfektion eines Gegenstandes eine die UV-C-Strahlung der zumindest einen Lichtquelle reflektierende Oberflächenstruktur aufweist, die ausgebildet ist, dass die reflektierte UV-C-Strahlung eine der Auflage zugewandte Seite des Gegenstandes bestrahlt. Dabei ist die Oberflächenstruktur derart ausgebildet, dass die der Auflage zugewandte Seite des abgelegten Gegenstandes zu einem tiefsten Punkt der Oberflächenstruktur beabstandet ist. Erfindungsgemäß ist vorgesehen, dass die Oberflächenstruktur als Wellenform ausgebildet ist. Die Wellenform weist sowohl Auflagebereiche als auch tiefere Bereiche auf, so dass die UV-C-Strahlung effizient reflektiert wird, um die der Auflage zugewandte Seite des Gegenstandes zu bestrahlen. Um zu ermöglichen, dass der Gegenstand aufliegt und dennoch seine der Auflage zugewandte Seite von der reflektierten UV-C-Strahlung erreicht wird, sind weiter erfindungsgemäß Erhebungen der Wellenform unterschiedlich hoch ausgebildet. Dabei bilden die höchsten Erhebungen die Auflagebereiche für den Gegenstand, wobei eine Anzahl der Auflagebereiche derart gewählt ist, dass der Gegenstand sicher aufliegt und ein Wackeln sowie eine dadurch bedingte Geräuschentwicklung weitestgehend ausgeschlossen werden können.

Mittels der Oberflächenstruktur der Auflage ist es möglich, die der Auflage zugewandte Seite des Gegenstandes im abgelegten Zustand desselben mit der UV-C-Strahlung zu bestrahlen, ohne dass es erforderlich ist, den Gegenstand umzudrehen. Die der Auflage zugewandte Seite liegt somit nicht im Schattenbereich und es ist dazu nicht erforderlich, eine Anzahl der UV-C-Strahlung emittierenden Lichtquellen zu erhöhen.

Dadurch ist es möglich, dass UV-C-Strahlung reflektiert wird und an die der Auflage zugewandte Seite des Gegenstandes gelangt. Somit kann also erreicht werden, dass ein Strahlengang reflektierter UV-C-Strahlung erzeugt wird, dass die reflektierte UV-C-Strahlung die der Auflage zugewandte Seite des Gegenstandes zumindest bereichsweise bestrahlt.

Die Oberflächenstruktur der Auflage ist in einer weiteren Ausführungsform ausgebildet, dass sich zumindest ein Teil der reflektierten UV-C-Strahlung in Bezug auf die der Auflage zugewandte Seite des abgelegten Gegenstandes verteilt. Insbesondere verteilt sich die reflektierte UV-C-Strahlung derart, dass die gesamte der Auflage zugewandte Seite des Gegenstandes erreicht und somit desinfiziert wird.

In einer möglichen Weiterbildung umfasst die Auflage eine reflektierende Trägerstruktur, welche die Oberflächenstruktur, insbesondere in Form der Wellenform, aufweist.

In einer alternativen Ausführungsform umfasst die Auflage eine Trägerstruktur, auf welcher eine Reflexionsschicht angeordnet ist, mittels welcher die mittels der Lichtquelle emittierte UV-C-Strahlung reflektiert wird, so dass diese verteilt wird und die die reflektierte UV-C-Strahlung die der Auflage zugewandte Seite des Gegenstandes zumindest bereichsweise bestrahlt. Dabei kann die Reflexionsschicht Silber und/oder Gold aufweisen.

Zudem sieht eine mögliche weitere Ausbildung vor, dass auf der Reflexionsschicht eine UV-C-Strahlung durchlassende Schutzschicht angeordnet ist, mittels welcher die Reflexionsschicht vor Beschädigungen, beispielweise Kratzern, geschützt werden kann. Ferner schützt die Schutzschicht die Reflexionsschicht gegenüber anderen äußeren Einflüssen und gegenüber Nässe.

Insbesondere sieht eine Ausführung vor, dass die Schutzschicht Siliziumdioxid umfasst, da dieses die UV-C-Strahlung weitestgehend passieren lässt, so die reflektierte UV-C-Strahlung die Unterseite des Gegenstandes erreicht.

Ausführungsbeispiele der Erfindung werden im Folgenden anhand von Zeichnungen näher erläutert.

Dabei zeigen:
- Fig. 1: schematisch ein Ablagefach mit unterhalb einer Auflage angeordneten Lichtquellen einer Desinfektionsvorrichtung,
- Fig. 2: schematisch eine Auflage mit einer Oberflächenstruktur und einer oberhalb der Auflage angeordneten Lichtquelle der Desinfektionsvorrichtung,
- Fig. 3: schematisch eine perspektivische Ansicht der Auflage mit Oberflächenstruktur und
- Fig. 4: schematisch ein Aufbau der Auflage.

Einander entsprechende Teile sind in allen Figuren mit den gleichen Bezugszeichen versehen.

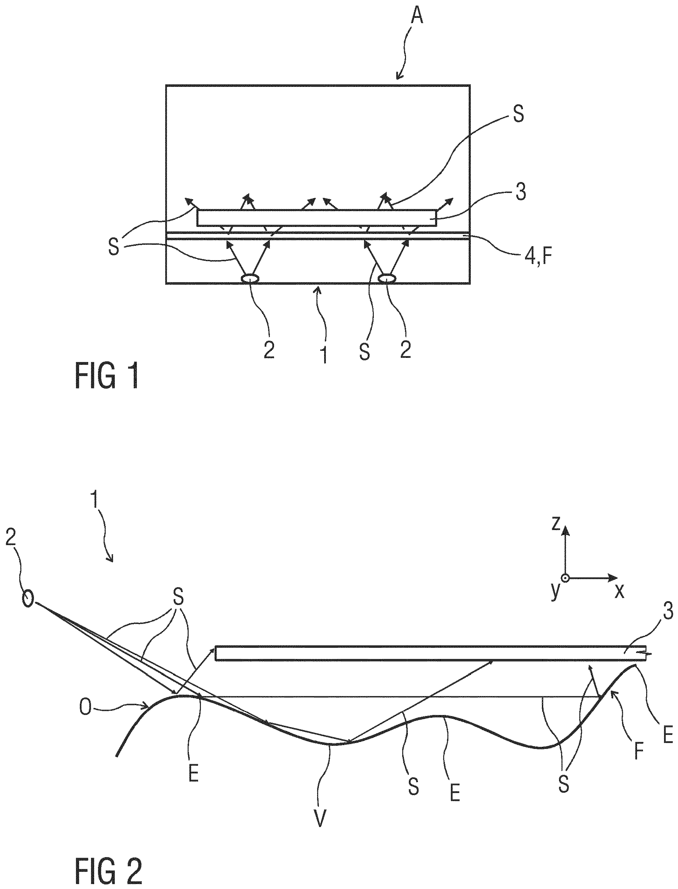

Figur 1 zeigt beispielhaft ein Ablagefach A für ein Fahrzeug, wobei in das Ablagefach eine Desinfektionsvorrichtung 1 integriert ist, die gemäß Figur 1 zwei UV-C-Strahlung S emittierende Lichtquellen 2 umfasst.

Das Ablagefach A dient zum Ablegen eines Gegenstandes 3, beispielsweise eines Smartphones, welche im abgelegten Zustand mittels der UV-C-Strahlung S desinfiziert wird.

Üblicherweise stellt eine Bestrahlung einer der Auflage zugewandte Seite des Gegenstandes 3 ein technisches Problem dar, da die der Auflage zugewandte Seite bei von oben auf den Gegenstand 3 emittierter UV-C-Strahlung S im Schattenbereich liegt. Gemäß dem vorliegenden Ausführungsbeispiel stellt die der Auflage zugewandte Seite des Gegenstandes 3 eine Unterseite desselben dar und wird im Folgenden als Unterseite bezeichnet.

Gemäß Figur 1 wird das technische Problem der Bestrahlung der Unterseite des Gegenstandes 3 dadurch gelöst, dass ein UV-C-Strahlung S durchlässiges Medium 4, welches insbesondere aus einer Quarzglasscheibe gebildet ist, eine Auflage F des Gegenstandes 3 bildet.

Die Lichtquellen 2 sind zwischen einem Boden des Ablagefaches A und dem die Auflage F bildenden Medium 4 angeordnet, mittels dessen die emittierte UV-C-Strahlung S gestreut wird, so dass sich diese verteilt.

Dadurch, dass die Lichtquellen 2 unterhalb des zu bestrahlenden Gegenstandes 3 angeordnet sind, wird die Unterseite bestrahlt, wobei zur derartigen Anordnung der Lichtquellen 2 ein erhöhter Bauraumbedarf besteht. Ein Bauraumbedarf zur Integrierung der Desinfektionsvorrichtung 1 in das Ablagefach A ist also erhöht, wodurch höhere Kosten sowie Nachteile in Bezug auf bauraumbedingte Verbaumöglichkeiten und eine Komplexität entstehen.

Um den Gegenstand 3 im in dem Ablagefach A abgelegten Zustand im Wesentlichen vollumfänglich mit der UV-C-Strahlung S bestrahlen und somit desinfizieren zu können, ohne, dass zumindest eine UV-C-Strahlung S emittierende Lichtquelle 2 unterhalb der Auflage F angeordnet ist, ist das Ablagefach A mit der integrierten Desinfektionsvorrichtung 1, wie im Folgenden beschrieben, ausgebildet.

Figur 2 zeigt eine Auflage F mit einer Oberflächenstruktur O, auf welcher der Gegenstand 3 abgelegt ist.

Dabei umfasst die Oberflächenstruktur O Erhebungen E und Vertiefungen V, so dass die Oberflächenstruktur O wellenförmig ausgebildet ist. Dabei bilden die höchsten Erhebungen E Auflagebereiche, auf denen der Gegenstand 3 aufliegt, wohingegen der Gegenstand 3 im abgelegten Zustand zu den Vertiefungen V, insbesondere zu deren tiefsten Punkten, beabstandet ist. Zudem ist die Auflage F reflektierend ausgeführt.

Alternativ oder zusätzlich kann die Oberflächenstruktur O der Auflage F auch eine andere geeignete Form aufweisen.

Die UV-C-Strahlung S emittierende Lichtquelle 2, bei welcher es sich insbesondere um eine Leuchtdiode handelt, ist in einem Eckbereich oder an einer Seitenwand des Ablagefaches A angeordnet. Die somit seitlich von oben kommende UV-C-Strahlung S wird durch die reflektierende Oberflächenstruktur O derart reflektiert, dass die UV-C-Strahlung S auf die Unterseite des Gegenstandes 3 gerichtet ist und diese erreicht.

Dazu ist die Oberflächenstruktur O derart ausgebildet, das die UV-C-Strahlung S bei Auftreffen auf die Auflage F nicht direkt senkrecht nach oben reflektiert wird sondern ein Teil der reflektierten UV-C-Strahlung S in einem vergleichsweise sehr flachen Winkel in einer Ebene verteilt wird. Somit ist es möglich, dass die Unterseite des Gegenstandes 3 nahezu vollständig von der reflektierten UV-C-Strahlung S bestrahlt wird.

Durch die wellenförmige Oberflächenstruktur O und/oder durch eine andere geeignete Oberflächenstruktur O, welche Auflagebereiche, d. h. Erhebungen E und Vertiefungen V aufweist, wird eine Verteilung der reflektierten UV-C-Strahlung S erreicht.

Die von Lichtquelle 2 in Form der Leuchtdiode emittierte UV-C-Strahlung S weist eine Vielzahl unterschiedlicher Abstrahlwinkel auf, wobei ein Strahlungskegel einer Leuchtdiode beispielsweise 120° beträgt.

Demzufolge ergeben sich durch die wellenförmige Oberflächenstruktur O der Auflage F eine Vielzahl von Eingangs- und Ausgangswinkeln, je nachdem, wo die UV-C-Strahlung S auf die Oberflächenstruktur O trifft. Ein solcher Punkt der Oberflächenstruktur O, an welchem die UV-C-Strahlung S reflektiert wird, wird als Reflexionspunkt R bezeichnet, wobei die Oberflächenstruktur O als Reflexionsgeometrie bezeichnet werden kann.

Aufgrund unterschiedlicher Höhen der Erhebungen E werden manche UV-C-Strahlen S direkt nach oben, in Richtung einer Hochachse z reflektiert, wohingegen sich andere UV-C-Strahlen S in horizontaler Richtung x verteilen und an einer anderen Stelle der Oberflächenstruktur O in Richtung der Hochachse z reflektiert werden.

Auch dreidimensional, z. B. in seitliche Richtung y, versetzte Erhebungen E unterschiedlicher Höhe tragen dazu bei, dass die UV-C-Strahlen S auf einer vergleichsweise großen Fläche verteilt werden. Diese sogenannten Hochpunkte müssen ebenfalls in seitlicher Richtung y ausgebildet werden, so dass die seitlich auftreffende UV-C-Strahlung S einen solchen Effekt erfährt.

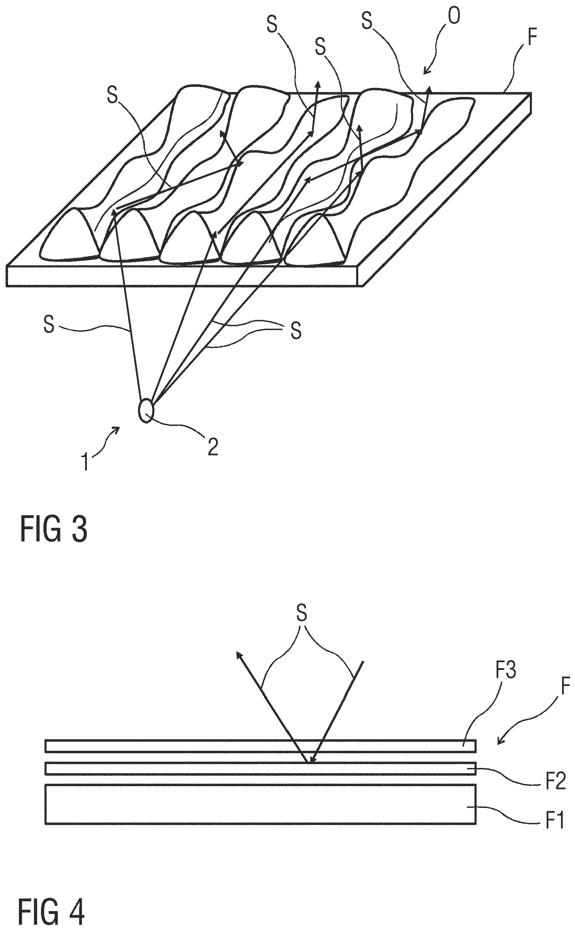

Das in Figur 2 gezeigte Ausführungsbeispiel stellt einen Ausschnitt der Auflage F und somit der Oberflächenstruktur O dar, wobei in Figur 3 eine perspektivische Ansicht der Auflage F mit der Oberflächenstruktur O gezeigt ist.

Aufgrund des Schattenbereiches des abgelegten Gegenstandes 3 erreichen die reflektierten UV-C-Strahlen S nur einen Teil der Oberflächenstruktur O, wobei sich die reflektierten UV-C-Strahlen S dennoch in horizontaler Richtung x ausbreiten und die Unterseite des Gegenstandes 3 nahezu vollständig bestrahlen.

Gemäß dem in Figur 2 gezeigten Ausführungsbeispiel können weitere Auflagebereiche der Oberflächenstruktur O, also der Auflage F in seitlicher Richtung y liegen.

Wie in Figur 3 gezeigt ist, erreichen die UV-C-Strahlen S aufgrund des Schattenbereiches des Gegenstandes 3 nur einen bestimmten Bereich der Reflexionsgeometrie, also der wellenförmigen Oberflächenstruktur O, wobei sich die UV-C-Strahlen S dennoch in horizontaler Richtung x ausbreiten und somit die Unterseite des Gegenstandes 3 fast vollständig bestrahlen können.

Figur 4 zeigt einen beispielhaften Aufbau der Auflage F ohne die ausgeformte Oberflächenstruktur O.

Die Auflage F als Trägerstruktur F1 kann beispielsweise aus einem vergleichsweise flexiblen thermoplastischen Elastomer gebildet sein, wobei auch weniger flexible Kunststoffe, wie z. B. Polypropylen, Polycarbonat und/oder Acrylnitril-Butadien-Styrol, verwendet werden können.

Auch eine Ausführung aus beispielsweise poliertem Aluminium, Edelstahl und/oder einem anderen reflektierenden Metall ist denkbar.

Ist die Trägerstruktur F1 der Auflage F aus einem nichtreflektierenden Kunststoff und/oder einem anderen nichtreflektierenden Material gebildet, ist es erforderlich auf die Trägerstruktur F1 eine Reflexionsschicht F2 aufzubringen, so dass auf diese auftreffende UV-C-Strahlen S reflektiert werden, um die Unterseite des abgelegten Gegenstandes 3 zu bestrahlen.

Beispielsweise kann als Reflexionsschicht F2 eine Aluminiumschicht, eine Goldschicht und/oder eine Silberschicht auf die Trägerstruktur F1, z. B. mittels eines vakuumbasierten Beschichtungsverfahren bzw. mittels einer Dünnschichttechnologie, aufgebracht werden.

Zum Schutz der Reflexionsschicht F2 gegen äußere Einflüsse und/oder Feuchtigkeit ist auf der Reflexionsschicht F2 eine Schutzschicht F3 angeordnet, welche in Bezug zumindest auf die UV-C-Strahlung S durchlässig ist. Da Quarzglas diese Eigenschaft aufweist, kann die Schutzschicht F3 Siliziumdioxid umfassen.

## Patentansprüche

1. Ablagefach (A) für ein Fahrzeug mit einer integrierten Desinfektionsvorrichtung (1) mit zumindest einer UV-C-Strahlung (S) emittierenden Lichtquelle (2) und mit einer Auflage (F) zur Auflage und Desinfektion eines Gegenstandes (3), die eine die UV-C-Strahlung (S) der zumindest einen Lichtquelle (2) reflektierende Oberflächenstruktur (O) aufweist, die ausgebildet ist, dass die reflektierte UV-C-Strahlung (S) eine der Auflage (F) zugewandte Seite des Gegenstandes (3) bestrahlt, wobei die der Auflage (F) zugewandte Seite des abgelegten Gegenstandes (3) zu einem tiefsten Punkt der Oberflächenstruktur (O) beabstandet ist, und wobei die Auflage (F) eine Trägerstruktur (F1) umfasst, auf welcher eine Reflexionsschicht (F2) angeordnet ist, **dadurch gekennzeichnet, dass** die Oberflächenstruktur (O) als Wellenform ausgebildet ist und dass Erhebungen (E) der Wellenform unterschiedlich hoch ausgebildet sind.

2. Ablagefach (A) nach Anspruch 1,
**dadurch gekennzeichnet, dass** die Oberflächenstruktur (O) ausgebildet ist, dass sich zumindest ein Teil der reflektierten UV-C-Strahlung (S) in Bezug auf die der Auflage (F) zugewandte Seite des abgelegten Gegenstandes (3) verteilt.

3. Ablagefach (A) nach einem der vorhergehenden Ansprüche,
**dadurch gekennzeichnet, dass** die Auflage (F) eine reflektierende Trägerstruktur (F1) umfasst.

4. Ablagefach (A) nach einem der vorhergehenden Ansprüche,
**dadurch gekennzeichnet, dass** auf der Reflexionsschicht (F2) eine zumindest UV-C-Strahlung (S) durchlassende Schutzschicht (F3) angeordnet ist.

5. Ablagefach (A) nach Anspruch 4,
**dadurch gekennzeichnet, dass** die Schutzschicht (F3) Siliziumdioxid umfasst.

## Claims

1. Storage compartment (A) for a vehicle, with an integrated disinfection device (1) having at least one light source (2) that emits UV-C radiation (S) and having a support (F) for supporting and disinfecting an object (3), which support has a surface structure (O) that reflects the UV-C radiation (S) of the at least one light source (2) and is designed such that the reflected UV-C radiation (S) irradiates a side of the object (3) facing the support (F), the side of the stored object (3) that faces the support (F) being spaced apart from a lowest point of the surface structure (O), and the support (F) comprising a carrier structure (F1) on which a reflection layer (F2) is arranged, **characterized in that** the surface structure (O) is designed as a wave shape **and in that** elevations (E) of the wave shape are of different heights.

2. Storage compartment (A) according to claim 1, **characterized in that** the surface structure (O) is designed such that at least part of the reflected UV-C radiation (S) is distributed with respect to the side of the stored object (3) facing the support (F).

3. Storage compartment (A) according to either of the preceding claims,
**characterized in that** the support (F) comprises a reflective carrier structure (F1).

4. Storage compartment (A) according to any of the preceding claims,
**characterized in that** a protective layer (F3) which lets through at least UV-C radiation (S) is arranged on the reflection layer (F2).

5. Storage compartment (A) according to claim 4,
**characterized in that** the protective layer (F3) comprises silicon dioxide.

## Revendications

1. Compartiment de rangement (A) pour un véhicule comportant un dispositif de désinfection (1) intégré comportant au moins une source de lumière (2) émettant un rayonnement UV-C (S) et comportant un support (F) pour le support et la désinfection d'un objet (3), lequel support présente une structure formant surface (O) réfléchissant le rayonnement UV-C (S) de l'au moins une source de lumière (2), laquelle structure formant surface est réalisée de sorte que le rayonnement UV-C (S) réfléchi irradie un côté de l'objet (3) orienté vers le support (F), dans lequel le côté de l'objet (3) rangé orienté vers le support (F) est espacé d'un point le plus profond de la structure formant surface (0), et dans lequel le support (F) comprend une structure porteuse (F1) sur laquelle est disposée une couche réfléchissante (F2), **caractérisé en ce que** la structure formant surface (O) est réalisée sous une forme ondulée **et en ce que** des bosses (E) de la forme ondulée sont réalisées à différentes hauteurs.

2. Compartiment de rangement (A) selon la revendication 1,
**caractérisé en ce que** la structure formant surface (O) est réalisée de sorte qu'au moins une partie du rayonnement UV-C (S) réfléchi se répartit par rapport au côté de l'objet (3) rangé orienté vers le support (F).

3. Compartiment de rangement (A) selon l'une des revendications précédentes, **caractérisé en ce que** le support (F) comprend une structure porteuse (F1) réfléchissante.

4. Compartiment de rangement (A) selon l'une des revendications précédentes, **caractérisé en ce qu'**une couche de protection (F3) laissant passer au moins le rayonnement UV-C (S) est disposée sur la couche réfléchissante (F2).

5. Compartiment de rangement (A) selon la revendication 4,
**caractérisé en ce que** la couche de protection (F3) comprend du dioxyde de silicium.
